# FASCICULE DE BREVET EUROPEEN

(11) **EP 1 080 744 B1**
(45) Date de publication et mention de la délivrance du brevet: **30.11.2005**
(21) Numéro de dépôt: 00402429.5
(22) Date de dépôt: 04.09.2000
(51) Int. Cl.: A61N 1/37

(54) **Stimulateur cardiaque implantable à ajustement automatique du niveau des impulsions de stimulation**
Implantierbarer Kardiostimulator mit automatischer Anpassung des Stimulationspulsniveaus
Implantable cardiac stimulator which automatically adjusts the level of stimulation pulses

(30) Priorité: 03.09.1999 FR 9911045
(43) Date de publication de la demande: 07.03.2001
(73) Titulaire: ELA MEDICAL, F-92541 Montrouge (FR)
(72) Inventeur: Casset, Cyril, 75010 Paris (FR); Bonnet, Jean-Luc, 92120 Montrouge (FR)
(74) Mandataire: Dupuis-Latour, Dominique

(56) Documents cités:
- EP-A- 0 334 681
- EP-A- 0 870 516
- US-A- 5 766 230
- US-A- 5 902 325

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement l'ajustement de l'amplitude (niveau de tension) des impulsions de stimulation au fil du temps.

Le niveau de stimulation des cavités cardiaques (ventriculaires ou auriculaires) est une valeur typiquement comprise entre 1,5 et 5 V (±10 %), réglable par pas de 0,25 V.

Cette amplitude doit être suffisamment élevée pour provoquer la dépolarisation de la cavité myocardique ; il faut cependant éviter des valeurs trop élevées pour ménager la durée de vie de la pile, car l'énergie de stimulation appliquée au myocarde, donc la consommation correspondante du dispositif, est proportionnelle au carré de l'amplitude (et également à la durée) de l'impulsion.

De plus, le seuil est une grandeur qui peut varier dans le temps, de sorte qu'il est souhaitable de pouvoir réévaluer à intervalles réguliers le niveau de l'amplitude de stimulation en opérant un test du seuil d'efficacité de la stimulation, appelé "test de capture". L'amplitude des impulsions de stimulation est ensuite ajustée sur la base du seuil de capture ainsi mesuré, typiquement à un niveau égal au double de la valeur du seuil mesuré, sous réserve d'un niveau minimum (typiquement 1,5 V) et d'un niveau maximum (typiquement 5 V) d'amplitude.

Le WO-A-93/02741 (Ela Médical) décrit ainsi un algorithme de test automatique du seuil de capture ventriculaire, mis en oeuvre dans le stimulateur de type *Talent* commercialisé par Ela Médical.

Un suivi clinique des patients équipés de cet appareil a révélé que dans certains cas l'algorithme de test de seuil automatique se fait leurrer, vraisemblablement en raison de fusions (stimulations intervenant de façon concomitante à un événement QRS spontané) survenant au moment du test. Ces anomalies se traduisent par une surévaluation de la valeur du seuil de capture par rapport au seuil réel du patient. Le réajustement de l'amplitude de stimulation étant opéré toutes les six heures, ce niveau excessif est maintenu pendant au moins six heures et, bien qu'il ne soit pas en lui-même dangereux, constitue une source de surconsommation et donc de réduction de la durée de vie de l'implant.

L'un des buts de l'invention est de remédier à cet inconvénient en proposant divers perfectionnements à l'algorithme de test automatique du seuil de capture, de manière à détecter ces anomalies susceptibles de survenir de manière erratique et d'en neutraliser les effets.

Plus précisément, le dispositif de l'invention, qui est du type décrit par le WO-A-93/02741 précité, c'est-à-dire comprenant des moyens de stimulation, pour délivrer au coeur des impulsions électriques de faible énergie pour le traitement des troubles du rythme cardiaque, ces impulsions présentant une amplitude et une durée prédéterminées, et des moyens d'ajustement de l'amplitude des impulsions de stimulation, comportant des moyens de mesure automatique du seuil de capture opérant à intervalles prédéterminés et délivrant en réponse une valeur de seuil de stimulation servant de base à l'ajustement de l'amplitude de stimulation.

Selon l'invention, les moyens d'ajustement d'amplitude comportent des moyens de validation de la valeur de seuil, propres à opérer un test de cohérence entre la dernière valeur de seuil mesurée et une valeur de référence fonction d'au moins l'une des valeurs de seuil correspondantes antérieurement mesurées, et à inhiber la modification du niveau d'amplitude de l'impulsion de stimulation en cas de résultat positif de ce test de cohérence.

Selon un certain nombre de caractéristiques subsidiaires avantageuses :
- ladite valeur de référence est l'une desdites valeurs de seuil antérieurement mesurées ;
- les moyens de validation produisent un résultat positif du test de cohérence lorsque l'écart entre la dernière et l'avant-dernière valeur de seuil est supérieur à un premier écart prédéterminé et lorsque l'écart entre l'avant-dernière et l'antépénultième valeur de seuil est inférieur à un second écart prédéterminé ;
- ledit premier ou second écart est égal à un pas de mesure du niveau de seuil, ou un multiple entier de ce pas ;
- le premier écart prédéterminé est supérieur ou égal au second écart prédéterminé ;
- en cas de résultat positif au test de cohérence, les moyens de validation déclenchent une réitération de la mesure du seuil de capture, le niveau de stimulation étant de préférence ajusté après réitération sur la base du plus bas des deux seuils mesurés, avant et après réitération ;
- dans ce dernier cas, le dispositif comprend en outre des moyens propres, lorsque la valeur du seuil de capture mesurée après réitération est supérieure à celle mesurée avant réitération, à réduire la durée du délai atrio-ventriculaire et réitérer la mise en oeuvre des moyens de mesure automatique du seuil de capture avec cette nouvelle valeur du délai atrio-ventriculaire.
- le dispositif comprend en outre des moyens propres, lorsque les moyens de mesure automatique du seuil de capture retournent un indicateur indiquant l'impossibilité de délivrer un résultat, à réduire la largeur des impulsions de stimulation et réitérer la mise en oeuvre des moyens de mesure automatique du seuil de capture avec cette nouvelle valeur de la largeur d'impulsion.

On va maintenant décrire en détail un exemple de mise en oeuvre de l'invention, en référence aux figures annexées.

La figure 1 est un synoptique d'assemblage des différents algorithmes illustrés sur les figures 2 à 5.

La figure 2 est un organigramme de l'algorithme de mesure de seuil et de contrôle de cohérence.

La figure 3 est un organigramme de l'algorithme de réitération de la mesure de seuil et de choix final de la valeur de seuil.

La figure 4 est un organigramme de l'algorithme d'ajustement par variation de la largeur d'impulsion.

La figure 5 est un organigramme de l'algorithme d'ajustement par variation du délai atrio-ventriculaire.

L'algorithme décrit dans le WO-A-93/02741 et mis en oeuvre dans l'appareil *Talent* d'ELA Médical prévoit de tester le seuil de capture du ventricule toutes les six heures, de manière automatique.

Ce test fournit trois résultats possibles :
1°) un indicateur "ÉCHEC", signifiant que, du fait de résultats aberrants à la calibration, le test ne peut fournir aucune valeur. La valeur de l'amplitude de stimulation est alors fixée automatiquement à la valeur maximale de 5 V, pour les six heures suivantes.
2°) un indicateur "RECHERCHE", signifiant que l'algorithme n'a pas pu se dérouler correctement. Les conditions de lancement du test n'étant pas réunies au cours des six heures précédentes (par exemple en raison d'un rythme trop rapide ou d'extrasystoles auriculaires), l'amplitude de stimulation est forcée à la valeur maximale de 5 V en attendant de pouvoir à nouveau lancer le test. Si cette situation perdure six heures de suite, alors l'algorithme produit l'indicateur " RECHERCHE" qui est enregistré dans le fichier de statistiques du stimulateur.
3°) un indicateur "OK", avec une valeur de mesure associée correspondant au dernier seuil de capture efficace trouvé par l'algorithme. Cette valeur est celle qui sera mémorisée par le stimulateur et qui servira à définir l'amplitude de stimulation ventriculaire pour les six prochaines heures.

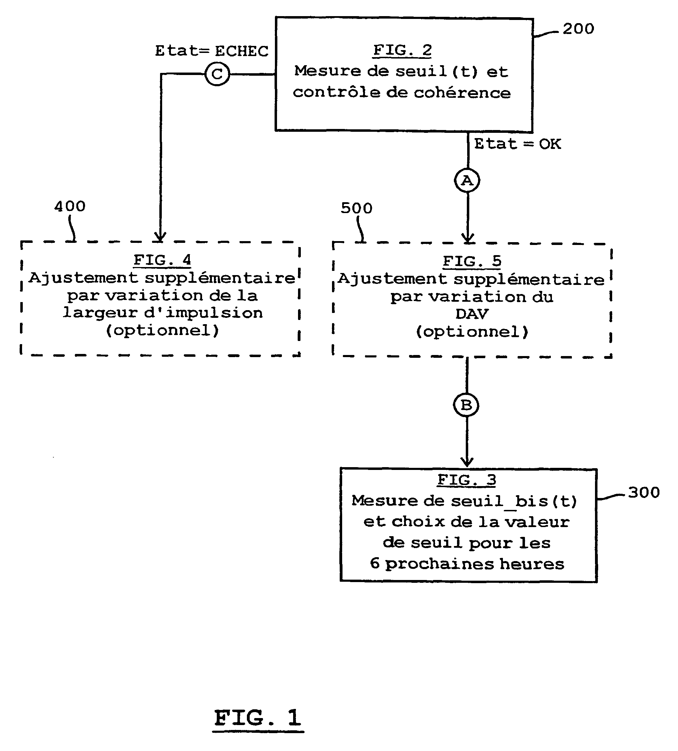

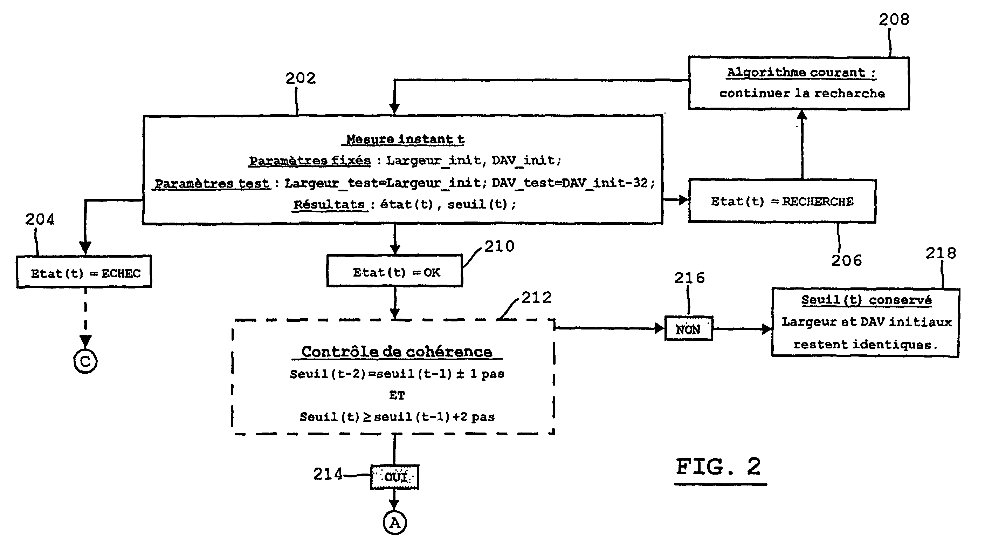

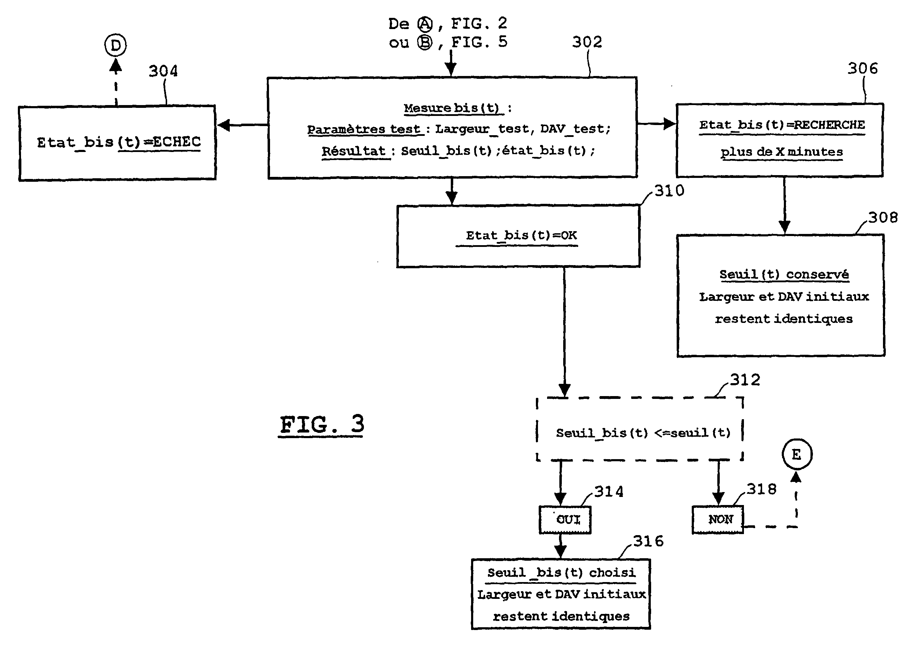

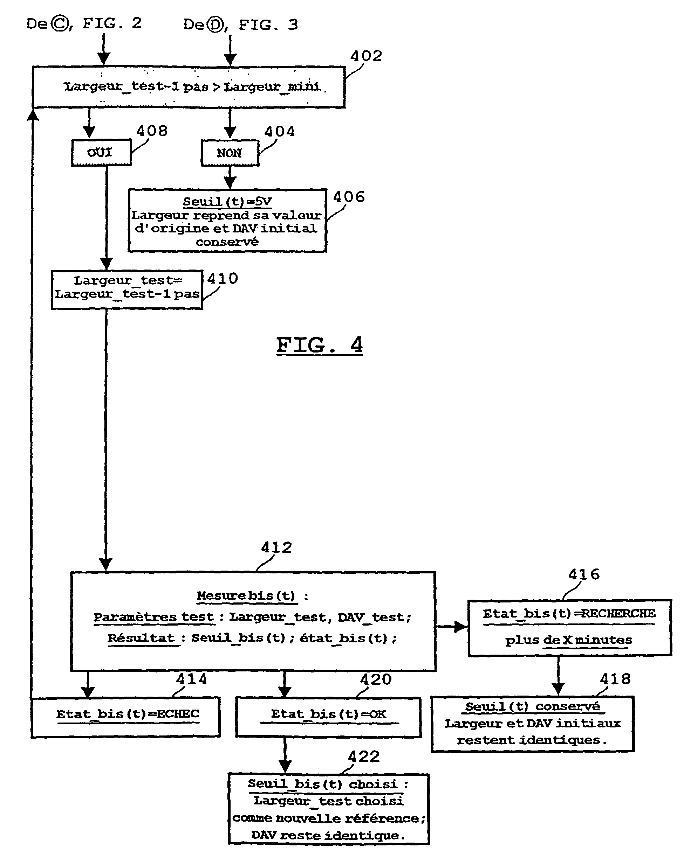

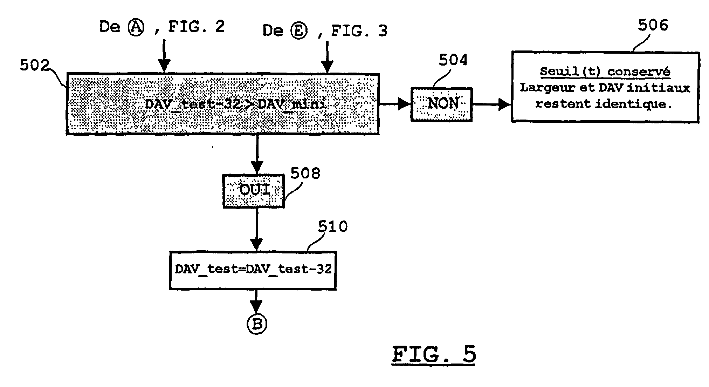

L'invention propose de compléter cet algorithme, sans le modifier, par un test de cohérence supplémentaire (algorithme 200 de la figure 1, décrit en détail figure 2), dont le principe consiste à confronter chaque nouvelle mesure de seuil à celles données par les tests précédents.

Si la valeur est jugée non cohérente, alors le test est relancé une seconde fois (algorithme 300 de la figure 1, décrit en détail figure 3), pour établir une valeur de seuil (nouvelle valeur ou ancienne valeur conservée) pour les six prochaines heures de fonctionnement du stimulateur, cette valeur étant établie sur la base d'un plus grand nombre de critères que dans l'algorithme antérieur, et donc avec un meilleur discernement et une meilleure efficacité sur le plan de la stimulation et, surtout, de la consommation de l'implant.

Dans un mode de mise en oeuvre subsidiaire avantageux, l'invention peut être perfectionnée en prévoyant en outre une mise en oeuvre avec variation de la largeur de l'impulsion (algorithme 400 de la figure 1, décrit en détail figure 4) et/ou du délai atrio-ventriculaire (algorithme 500 de la figure 1, décrit en détail figure 5) pendant la durée du test pour améliorer encore les qualités d'analyse de ce dernier.

Dans la suite et sur les figures, on utilisera les notations suivantes :
- État(t) : valeur "ÉCHEC", "RECHERCHE" ou "OK" de l'indicateur retournée par l'algorithme de test de seuil automatique.
- Seuil(t) : dernière valeur de seuil, c'est-à-dire la valeur mesurée par l'algorithme à l'instant t courant,
- Seuil(t-1) : avant-dernière valeur, mesurée par l'algorithme un test plus tôt que l'instant courant, c'est-à-dire normalement six heures plus tôt, ou moins si l'algorithme a produit un indicateur RECHERCHE.
- Seuil(t-2) : antépénultième valeur, c'est-à-dire valeur mesurée par l'algorithme deux tests plus tôt que l'instant courant, c'est-à-dire normalement douze heures plus tôt, ou moins si l'algorithme a produit un indicateur RECHERCHE.
- État_bis(t) : valeur de l'indicateur retournée une seconde fois, juste après la mesure de Seuil(t) (et non pas six heures après).
- Seuil_bis(t) : seuil mesuré à l'instant t une seconde fois, juste après la mesure de Seuil(t) (et non pas six heures après).
- DAV_init : délai atrio-ventriculaire (DAV) initial établi par l'appareil (programmé par le médecin, ou calculé par divers algorithmes qui ne font pas l'objet de l'invention), avant mise en oeuvre du test de capture.
- DAV_min : valeur minimale du DAV nécessaire pour pouvoir lancer un test de seuil.
- DAV_test : valeur du DAV utilisée pendant la durée du test de seuil.
- Largeur_init : largeur initiale de l'impulsion établie par l'appareil (programmée par le médecin, ou calculée par divers algorithmes qui ne font pas l'objet de l'invention), avant mise en oeuvre du test de capture ; la largeur des impulsions (dénommée quelquefois "longueur") est la durée, en millisecondes, d'application de l'impulsion électrique par les circuits de sortie du générateur d'impulsions.
- Largeur_min : valeur minimale de la largeur d'impulsion nécessaire pour pouvoir lancer un test de seuil.
- Largeur_test : valeur de la largeur d'impulsion utilisée pendant la durée du test de seuil.

### Mesure du seuil et contrôle de cohérence

On va tout d'abord décrire la manière dont le seuil est mesuré et dont est effectué le contrôle de cohérence, en référence à la figure 2.

La première étape, référencée 202, consiste à lancer à l'instant t un test automatique de seuil, par un procédé connu tel que celui décrit dans le WO-A-93/02741 précité. À cette étape 202, il peut être avantageux de réduire systématiquement la valeur du DAV, par exemple de 32 ms, pendant la durée du test pour réduire la probabilité de se trouver dans une situation de fusion (stimulation concomitante à une dépolarisation spontanée), le DAV retrouvant bien entendu en fin de test sa valeur initialement programmée.

Ce test délivre une valeur d'indicateur d'état État(t) et éventuellement une valeur de seuil Seuil(t).

Si État(t) retourne la valeur "ÉCHEC" (en 204), l'algorithme peut, de manière en elle-même connue, fixer automatiquement l'amplitude de stimulation à la valeur maximale de 5 V pour les six heures suivantes ou bien, de préférence et selon un perfectionnement avantageux et subsidiaire de l'invention, poursuivre le test en réitérant celui-ci avec une largeur d'impulsion réduite, comme on l'expliquera en référence à la figure 4.

Si État(t) retourne la valeur "RECHERCHE" (en 206), de manière classique l'algorithme gère la situation en tentant de relancer le test de capture automatique (en 208).

Si État(t) retourne la valeur "OK" (en 210), l'algorithme effectue alors un contrôle de cohérence (en 212), entre la dernière valeur Seuil(t) mesurée en 202, l'avant-dernière valeur Seuil(t-1) et l'antépénultième valeur Seuil(t-2) (un résultat "positif' de ce test signifie qu'il y a incohérence ; un résultat "négatif' signifie qu'il y a soit cohérence soit indétermination).

Plus précisément, si :
1°) les deux valeurs Seuil(t-1) et Seuil(t-2) sont proches, c'est-à-dire si elles ne sont pas éloignées l'une de l'autre de plus ou moins un pas de mesure (un pas valant typiquement 0,25 V), et
2°) l'avant-dernière valeur Seuil(t-1) est inférieure d'au moins deux pas à Seuil(t),
alors on considère (en 214) que la valeur Seuil(t) est surévaluée et nécessite d'être contrôlée à nouveau (bien entendu, les critères d'écart entre les seuils donnés ci-dessus ne sont donnés qu'à titre d'exemple, et peuvent être paramétrés et modifiés par le praticien).

Dans le cas contraire (en 216), la dernière valeur Seuil(t) est considérée comme cohérente avec l'avant-dernière et l'antépénultième valeur, et conservée (en 218) ; le niveau de l'amplitude de stimulation n'est donc pas modifié, non plus que la largeur d'impulsion et la valeur du DAV.

### Réitération du test

On va maintenant décrire la réitération du test, en référence à la figure 3. Si à l'étape 212 décrite ci-dessus on a déterminé que le test automatique de seuil devait être réitéré, le nouveau test (en 302) produit un nouvel indicateur d'état État_bis(t), et, éventuellement, une nouvelle valeur de seuil Seuil_bis(t).

Si État_bis(t) vaut "ÉCHEC" (en 304) on peut conserver Seuil(t) pour les six prochaines heures de la même manière que dans l'algorithme connu, ou bien, de préférence et selon un perfectionnement avantageux et subsidiaire de l'invention, poursuivre le test en réitérant celui-ci avec une largeur d'impulsion réduite, comme on l'expliquera en référence à la figure 4. Si (en 306) la valeur retournée par État_bis(t) est "RECHERCHE" pendant plus de X minutes (X étant un paramètre défini ou programmable), alors le seuil précédemment choisi (Seuil(t)) est conservé, de même que la largeur d'impulsion et le DAV initiaux (en 308). Il est en effet souhaitable de limiter la durée de l'étape de recherche de la valeur Seuil(t) afin d'éviter que l'algorithme ne délivre un résultat final "RECHERCHE" avec une consommation inutile pour un gain hypothétique - car dans ce cas la stimulation sera automatiquement forcée à la valeur maximale d'amplitude, comme indiqué plus haut.

Si le résultat de la réitération de la mesure du seuil à l'étape 302 donne pour État_bis(t) la valeur "OK" (en 310), alors on compare (en 312) la nouvelle valeur Seuil_bis(t) à la valeur Seuil(t) mesurée juste avant :
- Si Seuil_bis(t) < Seuil(t), on considère que Seuil(t) est une surévaluation ponctuelle aberrante et devant donc être éliminée.
- Si Seuil_bis(t) = Seuil(t), on considère que le seuil de stimulation s'est réellement élevé, de plus de deux pas en six heures, c'est-à-dire qu'il n'y a pas eu surévaluation aberrante, mais évaluation élevée correcte, à prendre en compte. Dans l'un ou l'autre cas (en 314), on conserve la valeur Seuil_bis(t) pour les six prochaines heures, de même que la largeur et le DAV initiaux (en 316).
- Si Seuil_bis(t) > Seuil(t) (en 318), on considère que le patient a présenté un rythme qui ne permet pas de conclure, la capture ayant été trouvée à Seuil(t) quelques cycles plus tôt. Dans ce cas, on peut conserver cette dernière valeur comme résultat final du test, Seuil_bis(t) étant considéré dans ce cas comme un résultat erroné causé par l'algorithme, à exclure. De préférence et selon un perfectionnement avantageux et subsidiaire de l'invention, on va chercher à poursuivre le test, en le réitérant sur la base d'un DAV réduit, comme on va le décrire en référence à l'algorithme de la figure 5.

### Modification de la largeur d'impulsion

L'algorithme de la figure 4 concerne un perfectionnement consistant à intégrer au test de seuil une variation de la largeur d'impulsion au cas où la valeur de État(t) retournée à l'étape 202, ou bien celle de État_bis(t) retournée à l'étape 302, est une valeur "ÉCHEC".

L'algorithme va alors jouer sur la largeur d'impulsion pour tenter de s'af franchir des limites du test de seuil ; en outre, si cette réduction s'avère bénéfique l'algorithme reprogrammera la largeur d'impulsion à la nouvelle valeur.

A l'étape 402, on vérifie tout d'abord si la réduction de largeur d'impulsion est possible, compte tenu de la valeur minimale Largeur_min programmée dans l'implant. Si cette réduction est impossible (en 404), alors on force l'amplitude de stimulation à 5 V, et la largeur ainsi que le DAV initiaux sont conservés (en 406).

Dans le cas contraire (en 408), la largeur d'impulsion est diminuée d'un pas (en 410) et on effectue immédiatement un nouveau test de seuil (en 412) avec cette nouvelle valeur Largeur_test, afin de déterminer une nouvelle valeur de l'indicateur d'état État_bis(t) et, éventuellement, une nouvelle valeur de seuil Seuil_bis(t), de la même manière qu'à l'étape 302 de la figure 3.

Si État_bis(t) vaut "ÉCHEC" (en 414), alors on va tenter, en retournant à l'étape 402, de réitérer une fois de plus la mesure de seuil sur la base d'une largeur d'impulsion encore plus réduite, bien entendu si cette réduction est possible.

Si la valeur de État_bis(t) retournée est "RECHERCHE" pendant une durée supérieure à X minutes (en 416), alors on conserve Seuil(t) comme valeur de seuil, de même que la largeur d'impulsion et le DAV initiaux (en 418). Si la valeur État_bis(t) retournée est "OK" (en 420), alors on choisit Seuil_bis(t) comme nouvelle valeur de seuil, le DAV initial étant conservé. En revanche, dans la mesure où la largeur d'impulsion modifiée a donné de meilleurs résultats au test, on choisi comme nouvelle référence pour les tests futurs la largeur d'impulsion modifiée. On notera que, dans ce cas où la largeur d'impulsion a été modifiée, la nouvelle valeur du seuil de capture devient la nouvelle et unique référence, les résultats précédents ne pouvant servir d'étalon car effectués à une largeur d'impulsion différente.

### Modification de la durée du délai atrio-ventriculaire

L'algorithme de la figure 5 concerne un perfectionnement consistant à intégrer au test de seuil une variation du DAV.

En effet, lorsque le test de l'étape 312 donne un résultat négatif (en 318), c'est-à-dire lorsque, dans le cas de deux mesures rapprochées du seuil, la seconde mesure donne une valeur supérieure à la première, il peut s'agir d'une situation de fusion (stimulation concomitante à une dépolarisation spontanée) ; la réduction du DAV offre alors à l'algorithme une chance d'anticiper la dépolarisation spontanée et donc d'être certain que le test s'effectue sur une stimulation seule, sans fusion.

Pour ce faire, l'algorithme tente de réduire le DAV d'une valeur donnée, par exemple de 32 ms.

On vérifie d'abord (en 502), que cette réduction du DAV est possible, c'est-à-dire que l'on n'a pas atteint le DAV minimal DAV_min programmé dans l'implant. Si la réduction est impossible (en 504), alors on conserve la valeur de Seuil(t), de même que la largeur d'impulsion et le DAV initiaux (en 506).

Si, en revanche, la réduction est possible (en 508), alors on réduit le DAV (en 510) d'une valeur prédéterminée, par exemple de 32 ms, et l'on réitère la mesure de Seuil_bis(t) de la même façon que précédemment (comme expliqué en référence à la figure 3), mais sur la base de cette nouvelle valeur DAV_test, et ainsi de suite.

On notera que, après exécution complète de l'algorithme, le DAV reprend toujours sa valeur initiale, sa modification n'intervenant que pendant la durée du test. Il en est autrement de la largeur d'impulsion qui peut être modifiée non seulement pendant le test, mais dont la valeur de référence peut être différente en fin d'algorithme de celle qu'elle avait au début.

## Revendications

1. Un dispositif médical actif implantable, notamment un stimulateur cardiaque, défibrillateur ou cardioverteur, comprenant :
- des moyens de stimulation, pour délivrer au coeur des impulsions électriques de faible énergie pour le traitement des troubles du rythme cardiaque, ces impulsions présentant une amplitude et une durée prédéterminées, et
- des moyens d'ajustement de l'amplitude des impulsions de stimulation, comportant des moyens de mesure automatique du seuil de capture opérant à intervalles prédéterminés et délivrant en réponse une valeur de seuil de stimulation servant de base à l'ajustement de l'amplitude de stimulation,
dispositif **caractérisé en ce que** les moyens d'ajustement d'amplitude comportent des moyens (20, 30, 50) de validation de la valeur de seuil, propres à opérer un test de cohérence entre la dernière valeur de seuil mesurée et une valeur de référence fonction d'au moins l'une des valeurs de seuil correspondantes antérieurement mesurées, et à inhiber la modification du niveau d'amplitude de l'impulsion de stimulation en cas de résultat positif de ce test de cohérence, un résultat positif de ce test signifiant qu'il y a incohérence.

2. Le dispositif de la revendication 1, dans lequel ladite valeur de référence est l'une desdites valeurs de seuil antérieurement mesurées.

3. Le dispositif de la revendication 2, dans lequel les moyens de validation produisent un résultat positif du test de cohérence lorsque l'écart entre la dernière et l'avant-dernière valeur de seuil est supérieur à un premier écart prédéterminé et l'écart entre l'avant-dernière et l'antépénultième valeur de seuil est inférieur à un second écart prédéterminé.

4. Le dispositif de la revendication 3, dans lequel ledit premier ou second écart est égal à un pas de mesure du niveau de seuil, ou un multiple entier de ce pas.

5. Le dispositif de la revendication 3, dans lequel le premier écart prédéterminé est supérieur ou égal au second écart prédéterminé.

6. Le dispositif de la revendication 1, dans lequel, en cas de résultat positif au test de cohérence, les moyens de validation déclenchent une réitération de la mesure du seuil de capture.

7. Le dispositif de la revendication 6, dans lequel, après réitération, le niveau de stimulation est ajusté sur la base du plus bas des deux seuils mesurés, avant et après réitération.

8. Le dispositif de la revendication 6, comprenant en outre des moyens propres, lorsque la valeur du seuil de capture mesurée après réitération est supérieure à celle mesurée avant réitération, à :
- réduire la durée du délai atrio-ventriculaire, et
- réitérer la mise en oeuvre des moyens de mesure automatique du seuil de capture avec cette nouvelle valeur (DAV_test) du délai atrio-ventriculaire.

9. Le dispositif de la revendication 1, comprenant en outre des moyens propres, lorsque les moyens de mesure automatique du seuil de capture retournent un indicateur (ÉCHEC) indiquant l'impossibilité de délivrer un résultat, à :
- réduire la largeur des impulsions de stimulation, et
- réitérer la mise en oeuvre des moyens de mesure automatique du seuil de capture avec cette nouvelle valeur (Largeur_test) de la largeur d'impulsion.

## Patentansprüche

1. Aktive, implantierbare medizinische Vorrichtung, insbesondere ein Herzschrittmacher, Defibrillator oder Kardioverter, die aufweist:
- Stimulationsmittel, um an das Herz elektrische Impulse schwacher Energie zur Behandlung von Herzrhythmusstörungen zu liefern, wobei diese Impulse eine vorbestimmte Amplitude und Dauer aufweisen, und
- Mittel zum Einstellen der Amplitude der Stimulationsimpulse, die Mittel zum automatischen Messen des Erfassungsschwellenwerts aufweisen, die zu vorbestimmten Intervallen arbeiten und als Antwort einen Stimulationsschwellenwert liefern, der als Basis für die Einstellung der Stimulationsamplitude dient,
wobei die Vorrichtung **dadurch gekennzeichnet ist, dass** die Mittel zum Einstellen der Amplitude Mittel (20, 30, 50) zur Validierung des Schwellenwertes aufweisen, die dazu geeignet sind, einen Kohärenztest zwischen dem letzten gemessenen Schwellenwert und einem Referenzwert abhängig von zumindest einem der zuvor gemessenen entsprechenden Schwellenwerte durchzuführen und die Änderung des Amplitudenniveaus des Stimulationsimpulses im Falle eines positiven Ergebnisses dieses Kohärenztestes zu sperren, wobei ein positives Ergebnis dieses Testes bedeutet, dass eine Inkohärenz vorliegt.

2. Vorrichtung gemäß Anspruch 1, bei der der Referenzwert einer der zuvor gemessenen Schwellenwerte ist.

3. Vorrichtung gemäß Anspruch 2, bei der die Mittel zur Validierung ein positives Kohärenztestergebnis erzeugen, wenn der Abstand zwischen dem letzten und dem vorletzten Schwellenwert größer als ein erster vorbestimmter Abstand ist und der Abstand zwischen dem vorletzten und dem vorvorletzten Schwellenwert kleiner als ein zweiter vorbestimmter Abstand ist.

4. Vorrichtung gemäß Anspruch 3, bei der der erste oder zweite Abstand gleich einem Messschritt des Schwellenwertniveaus oder einem ganzen Vielfachen dieses Schrittes ist.

5. Vorrichtung gemäß Anspruch 3, bei der der erste vorbestimmte Abstand größer oder gleich dem zweiten vorbestimmten Abstand ist.

6. Vorrichtung gemäß Anspruch 1, bei der im Falle eines positiven Ergebnisses des Kohärenztestes die Mittel zur Valdierung eine Wiederholung der Messung des Erfassungsschwellenwerts auslösen.

7. Vorrichtung gemäß Anspruch 6, bei der nach Wiederholung das Stimulationsniveau auf der Basis des niedrigsten der beiden vor und nach der Wiederholung gemessenen Schwellenwerte eingestellt wird.

8. Vorrichtung gemäß Anspruch 6, die außerdem eigene Mittel aufweist, um, wenn der gemessene Erfassungsschwellenwert nach der Wiederholung größer als der vor der Wiederholung gemessene ist:
- die Dauer der atrio-ventrikulären Verzögerung zu reduzieren, und
- das Ausführen der Mittel zum automatischen Messen des Erfassungsschwellenwerts mit diesem neuen Wert (DAV_test) der atrioventrikulären Verzögerung zu wiederholen.

9. Vorrichtung gemäß Anspruch 1, die außerdem eigene Mittel aufweist, um, wenn die Mittel zum automatischen Messen des Erfassungsschwellenwertes einen Indikator (ECHEC) zurücksenden, der anzeigt, dass es unmöglich ist, ein Ergebnis zu liefern:
- die Breite der Stimulationsimpulse zu reduzieren, und
- das Ausführen der Mittel zum automatischen Messen des Erfassungsschwellenwertes mit diesem neuen Wert (Largeur_test) der Impulsbreite zu wiederholen.

## Claims

1. An active implantable medical device, in particular a cardiac pacemaker, defibrillator or cardioverter, comprising:
- stimulation means for delivering to the heart stimulation pulses of low energy for treating cardiac rate disorders, said pulses having a predetermined amplitude and duration, and
- means for adjusting the amplitude of the stimulation pulses, comprising means for automatically measuring the capture threshold at predetermined interval and delivering in response a stimulation threshold value used as a basis for the adjustment of the stimulation amplitude,
said device being **characterised in that** the amplitude adjustment means comprises means (20, 30, 50) for validating the threshold value, suitable to perform a coherence test between the last measured threshold value and a reference value which is a function of at least one of the previously measured corresponding threshold values, and to inhibit the modification of the amplitude level of the stimulation pulse in case of positive result of said coherence test, a positive result of said coherence test meaning that coherence is missing.

2. The device of claim 1, wherein said reference value is one of said previously measured threshold values.

3. The device of claim 2, wherein the validating means provides the positive result of the coherence test when the difference between the last and the penultimate value of the threshold is greater than a first predetermined difference and the difference between the penultimate and the ante-penultimate value of the threshold is less than a second predetermined difference.

4. The device of claim 3, wherein each of said first and second predetermined differences is equal to a measurement step of the threshold value, or an integer multiple of said step.

5. The device of claim 3, wherein the first predetermined difference is greater than or equal to the second predetermined difference.

6. The device of claim 1, wherein in case of positive result to the coherence test, the validating means initiate a reiteration of the capture threshold measurement.

7. The device of claim 6, wherein after reiteration the stimulation level is adjusted on the basis of the lowest of the two thresholds as measured before and after reiteration.

8. The device of claim 6, further comprising means adapted, when the value of the capture threshold as measured after reiteration is greater than the one as measured before reiteration, to:
- reduce the duration of the atrio-ventricular delay, and
- reiterate the operation of the means for automatically measuring the capture threshold with said modified value (DAV_test) of the atrio-ventricular delay.

9. The device of claim 1, further comprising means adapted, when the means for automatically measuring the capture threshold sends back an indicator (ÉCHEC) stating the impossibility of issuing a result, to:
- reduce the width of the stimulation pulses, and
- reiterate the operation of the means for automatically measuring the capture threshold with said modified value (Largeur_test) of the width of the stimulation pulses.
